# EUROPEAN PATENT SPECIFICATION

(11) **EP 2 678 006 B1**
(45) Date of publication and mention of the grant of the patent: **30.12.2015**
(21) Application number: 12706778.3
(22) Date of filing: 24.02.2012
(51) Int. Cl.: A61K 9/00, A61K 9/14, A61K 9/16, A61K 31/21, A61K 47/14, A61K 47/26, A61P 9/10, B65D 85/00, A61J 1/03

(54) **PACKAGING OF SOLID PHARMACEUTICAL PREPARATIONS CONTAINING THE ACTIVE SUBSTANCE GLYCERYL TRINITRATE**
VERKAPSELUNG VON FESTEN PHARMAZEUTISCHEN ZUBEREITUNGEN MIT DEM WIRKSTOFF GLYCERYLTRINITRAT
CONDITIONNEMENT DE PRÉPARATIONS PHARMACEUTIQUES SOLIDES CONTENANT LA SUBSTANCE ACTIVE TRINITRATE DE GLYCÉRYLE

(30) Priority: 25.02.2011 DE 102011012491
(43) Date of publication of application: 01.01.2014
(73) Proprietor: G. Pohl-Boskamp GmbH & Co. KG, 25551 Hohenlockstedt (DE)
(72) Inventor: ZIMMECK, Thomas, 25551 Hohenlockstedt (DE); UECK, Henning, 25524 Bekmünde (DE); GEHRICKE, Julia, 24106 Kiel (DE)
(74) Representative: Hamm, Volker
(86) International application number: PCT/EP2012/000802
(87) International publication number: WO 2012/113563

(56) References cited:
- WO-A1-03/066472
- WO-A2-01/68062
- DANIEL BANES: "Deterioration of nitroglycerin tablets", JOURNAL OF PHARMACEUTICAL SCIENCES, vol. 57, no. 5, 1 May 1968 (1968-05-01), pages 893-894, XP55027097, ISSN: 0022-3549, DOI: 10.1002/jps.2600570545 cited in the application
- M. J. PIKAL ET AL: "Polymer sorption of nitroglycerin and stability of molded nitroglycerin tablets in unit-dose packaging", JOURNAL OF PHARMACEUTICAL SCIENCES, vol. 66, no. 9, 1 September 1977 (1977-09-01), pages 1293-1297, XP55027290, ISSN: 0022-3549, DOI: 10.1002/jps.2600660922
- DATABASE CAPLUS [Online] CHEMICAL ABSTRACTS SERVICE, COLUMBUS, OHIO, US; 2004, CHEN, BAOXI ET AL: "Effect of acrylonitrile-butadiene rubber on nitroglycerin migration from propellant to EPDM inhibitor", XP002675994, retrieved from STN Database accession no. 2004:826842
- "Barex Resins", INEOS Barex, USA , 2006, XP002675995, Retrieved from the Internet: URL:http://www.ineosbarex.com/files/upload /Ineos%20Barex%20Brochure.pdf [retrieved on 2012-05-15]

## Description

The present invention relates to solid pharmaceutical preparations containing volatile active substances in the form of one or more individual doses packaged in specific composite films or blister foils with improved storage stability. In particular, the present invention relates to solid pharmaceutical preparations for oral or oromucosal administration containing the active substance glyceryl trinitrate (nitroglycerin, referred to below in abbreviated form as GTN) in the form of pre-dosed granules or in the form of tablets, which are packaged individually in a stick pack, a four-sided sealed pouch or a blister, respectively.

GTN is an active substance which is used in the treatment of attacks of angina pectoris, among other uses, whereby it is especially used in emergency situations, in which the pharmaceutical form must enable a rapid onset of action. Within this framework sublingual administration has proven very effective with rapid uptake of the active substance and quick relief of symptoms. In addition to sublingual sprays and chewable capsules, tablets for oromucosal, i.e. sublingual or buccal administration with rapid onset of action have proven to be especially effective pharmaceutical forms for the outpatient sector.

Sprays for sublingual administration, which are used to spray the active substance-containing dose underneath the tongue, provide for a direct and rapid application of the dissolved active substance over a large area of the highly resorbent oral mucosa.

However, affected patients are required to carry a relatively voluminous spray bottle around with them at all times in order to ensure immediate access to the medicinal product in emergencies and enable a rapid administration of the GTN. Alternatively, chewable capsules containing the active substance as an oily solution can be carried around as individual doses in blisters. However, a portion of the active ingredient, which is released by tearing open the capsules with the teeth, never reaches the sublingual area, its absorption is delayed or it is lost through swallowing. Sublingual tablets and oral fast dissolving films (also referred to as edible films) represent a further alternative to spray solutions and chewable capsules because they can be placed directly under the tongue to rapidly release the active substance.

With a vapor pressure value of 0.25 Pa at 20 °C, GTN is volatile in liquid as well as in solid formulation. Thus, it has been observed that tablets lose some of the active substance merely by opening the package to remove some of them, resulting in a limited shelf life.

Up to now commercially available GTN tablets have been filled into glass bottles, which are relatively inert against interactions with the filled product. In addition, they are transparent and protect the contents from external influences. However, due to their low shatter resistence glass containers must be handled with a certain level of caution when carried around by patients. In addition, due to the small size of the tablets the removal of a single tablet can be difficult in an emergency situation, when patients can easily drop the container. Moreover, it is not practical to package individual doses in glass containers and the patients are required to carry around the entire original packaging. For this reason, there is the long felt unmet need for solid and stable pharmaceutical forms for GTN, which are easy for patients to carry around in single-dose form, e.g. in a wallet or jacket pocket, and which simultaneously ensure sufficient stability, simple administration, and rapid onset of action.

So-called stick packs or sachets (four-sided sealed pouches) represent an alternative configuration for individual doses, which are becoming increasingly common in the pharmaceutical industry. Stick packs are typically manufactured using composite films consisting of several layers, whereby each individual layer of film performs a specific task (barrier, stiffness, impact resistance, sealing properties, etc.). Here, the barrier layer performs the task of protecting the package contents against substance loss and external contamination. A support layer stabilizes the packaging, enabling it to withstand mechanical stresses, while the sealing layer serves mainly to seal the film package. It is usually made of a thermoplastic polymer and is located on the side of the composite film facing the filling material. Stick packs can be manufactured to contain the pharmaceutical preparation as flowable granules or powder and enable both comfortable transport of the medicinal product as well as simple, easy and reproducible dosing, which is especially significant in emergency situations. However, GTN granules - with their substantially larger total surface area as compared to tablets - pose an even greater challenge with respect to the stabilization of the preparation in any type of packaging configuration including stick packs. In the case of GTN, the highest possible storage stability is especially critical to enable the patient to carry around a single dose such as, but not limited to a stick pack. However, carrying it around in the breast pocket of a shirt, for example, can subject it to significant temperature increases and associated stress conditions, which may violate the recommended storage conditions for GTN preparations.

Another kind of a pharmaceutical preparation that is available in the form of a single dose applicable to the oral mucosa is an oral fast dissolving film (OFDF). This delivery system consists of a slim oral strip, which can be easily placed on the oromucosal tissue. Saliva instantly wets the OFDF, which rapidly hydrates and adheres onto the site of application followed by its rapid disintegration and release of the contained drug substance.

It is known from the prior art that GTN has a high affinity for organic materials. Thus, according to Schou, S. A. (Pharmaceutica acta Helvetiae, 34, 1959, 309-330), the use of organic materials, such as cork, cotton, paper, and especially rubber and polyethylene to store GTN-containing tablets must be avoided. Banes (Journal of Pharmaceutical Sciences, 57, 1968, 893) reported GTN loss in tablets packaged individually in aluminum foil films containing a sealing layer made of polyethylene. Following storage, the tablets were found to have less than 10% of the active substance they originally contained, while the majority of the GTN was found in the sealing layer of the packaging material. This effect was attributed to the volatility of GTN at room temperature as well as the high permeability of the plastic for GTN. Polypropylene and polyvinyl chloride appear to behave similarly to a polyethylene, which was discovered to contain 84 % of the active substance originally contained in the tablets. Edelman et al. (Journal of the American Pharmaceutical Society, NS11, 1971, 30-33) reported on studies in which the stability of GTN tablets was tested based on the material of the storage container. Here, several tablets were packaged in containers made of glass or polystyrene and stored for 60 days at 25 °C and 50 °C, respectively. The results revealed a significant drop in the GTN content of the tablets packaged in plastic containers, while the content of the tablets stored in glass containers remained essentially stable. Tablets packaged individually in blisters and stored under the conditions described above were found to have an even lower GTN content than the tablets stored together in plastic containers. GTN loss occurred regardless of whether the tablets were packaged in plastic-aluminum blisters or in aluminum-aluminum blisters. As a result, storage of GTN tablets in blisters was not recommendable and GTN tablets for sublingual administration are still not available on the market in single-dose units packaged in blisters. Taken together it is common knowledge of pharmaceutical experts that solid preparations containing GTN as active substance can not be packaged in plastic materials.

Moreover, Pikal et al. (Journal of Pharmaceutical Sciences, 66, 1977, 1295) demonstrate the different, predominantly considerable affinity of various polymers (vinyls, low density polyethylene, high density polyethylene, ionomers) to GTN, which limit their use in primary packaging of GTN compositions. Chen et al. (Database Caplus [online], CAS, Columbus Ohio, US, 2004, database accession no. 2004:826842) investigated the effect of the acrylonitrile-butadiene content in EPDM rubber inhibitor formulations on the inhibitor's performance of resisting nitroglycerin migration using the intermediate layer method. It was demonstrated that the content of acrylonitrile-butadiene in the EPDM rubber inhibitor had significant impact on the inhibitors performance.

International guidelines require that medicinal products must remain stable for six months at 40 °C and a relative humidity of 75 % and - depending on climate zone - for the entire shelf life at 30 °C and a relative humidity of 65 % or at 25 °C and a relative humidity of 60 %. For logistic and marketing reasons, manufacturers usually aim for a shelf life of at least two years. However, due to the volatility of the active substance and its affinity for plastics, this shelf life is rarely achieved in the case of GTN-containing preparations. There thus continues to be a need for stable GTN-containing preparations having a sufficient shelf life, which are able to be provided in a single dose.

A primary packaging material suitable for the production of individual doses, e.g. in the form of stick packs, must be flexible and sufficiently stable in order to avoid being destroyed during transport. In addition, the packaging material must be inert and impervious to the active substance as well as to impurities, which can reach the preparation from the outside.

A series of materials is available in the form of composite films for the production of stick packs, sachets and blisters. Among these, aluminum composite films are especially suited for the packaging of pharmaceutical preparations which should be protected from external influences. They exhibit the following layer structure:
Paper - Aluminum - Sealing medium
Polyethylene terephthalate (PET) - Aluminium - Sealing medium

Both, paper as well as PET, serve as the support layer and increase the mechanical stability of the packaging, while aluminum serves as the barrier layer. or PET layer is located on the external surface of the packaging and can be imprinted for their identification. The surface of the aluminum film coming into contact with the filling material is provided with a sealing medium. Sealing materials differ in certain physico-chemical properties, e.g. the softening temperature and a possible interaction with the components of the pharmaceutical preparation. Available materials include composite films with polyethylene (PE), polypropylene (PP), low-density polyethylene (LDPE) or polyacrylonitrile (PAN) as sealing layer. The goals of an effective sealing process are 100 % tightness of the packages, maximum throughput and protection of the pharmaceutical preparation from thermal stress. The sealant strongly influences these parameters and thus the industrial applicability of the process.

In the sense of this invention the term PAN comprises polyacrylonitrile as well as copolymers of acrylonitrile units and one or more other monomers (AN-copolymers) such as acrylonitrile-methylacrylate copolymer.

In addition to aluminum-aluminum blisters, blisters consisting of an aluminum push-through foil and a thermoplastic film are also available for packaging tablets. Suitable thermoplastic films include thermoplastic composite films, which typically consist of multiple layers of different plastics, such as PE, PVC, PP, LDPE or PAN. The mentioned packaging materials are also suitable for other kinds of dosage forms available for individual doses such as powders and orally fast dissolving films.

Examples of composite films with PAN at the side facing the product are available from the international patent applications WO 01/068062 and WO 03/066472, which describe packaging materials useful for transdermal drug delivery systems containing chiral drugs such as methylphenidate, and child resistant sachets having a sufficient tear resistance, respectively. In addition, WO 03/066472 mentions that acrylonitrile-methylacrylate copolymer resins (e.g. Barex^{®} resins) are more difficult to be bonded by melt sealing than other materials, but have advantageous flavor barrier properties. Advantageous gas barrier properties of Barex^{®} resins are also known from the corresponding product information ("Barex^{®} Resins", INEOS Barex, USA, ***2006***, retrieved from the Internet: URL:http://www.ineosbarex.com/files/upload/Ineos%20Barex%20Brochure.pdf on 2012-05-15).

Thus, the object of the present invention is to provide a solid pharmaceutical preparation containing GTN for oral and oromucosal (such as sublingual or buccal) administration in the form of individually packaged single doses having good storage stability.

This object is attained by the provision of a stable medicament comprising a solid pharmaceutical preparation containing the active substance GTN for oral or oromucosal administration in the form of one or more individual doses, wherein the one or more individual doses are packaged in aluminium composite films or thermoplastic blister films, which contain a layer comprising PAN on the side facing the preparation. Thus, the present invention exploits the finding that against the expectation of a person skilled in the art thermoplastic materials could be found that exhibit minimal interaction with GTN.

Within the framework of the investigations upon which the present invention is based, it was surprisingly found that solid GTN-containing preparations remain stable for the required time period if they are packaged in composite films containing a layer comprising PAN on the surface facing the product. Preferred are aluminum composite films containing a sealing layer comprising an acrylonitrile-methylacrylate copolymer, while aluminium composite films containing a sealing layer made of an impact-modified acrylonitrile-methylacrylate copolymer are particularly preferred. Such barrier plastics are marketed, among others, under the commercial names Barex^{®} 210 or Barex^{®} 218. The preparations according to the invention are packaged as individual doses which remain stable for a storage period of at least 6 months at 40 °C and 75 % relative humidity.

The preferred pharmaceutical preparation is an oromucosal, solid, GTN-containing preparation, preferably a GTN-containing, flowable powder or flowable granules, respectively, for sublingual administration, which may optionally be pressed into a sublingual tablet.

The pharmaceutical preparation contains 0.05 to 2 weight % GTN; GTN concentrations of 0.10, 0.20, 0.25, 0.30, 0.40, 0.50, 0.60, 0.70, 0.80, 1.0, 1.2, 1.4, 1.6 or 1.8 weight % are especially preferred. Unless otherwise stated, all weight percentages are based on the total weight of the preparation.

The pharmaceutical preparation according to the invention may contain one or more other pharmaceutical excipients, which support sublingual release of the active substance and which are selected from water-soluble mono-, di-, and polysaccharides, as well as their alcohols. These excipients are especially selected from fructose, glucose, isomalt, lactose, maltitol, maltose, mannitol, saccharose, sorbitol, trehalose and xylitol, and mixtures thereof. These excipients are preferably present in a total content of 90 to 98 weight %. In the case of mixtures, the concentration of each individual substance is 20 to 70 weight %, whereby the preferred contents include 25, 30, 35, 40, 45, 50, 55, 60 or 65 weight %.

The preparation according to the invention may optionally be provided with a coating. In addition, the preparation according to the invention may contain other excipients, such as flavoring agents. Flavoring agents are used especially in the case of preparations for oral or sublingual administration in order to increase acceptance among patients. According to the invention, they are preferably used at concentrations of 0.01 to 3.0 weight %, whereby the especially preferred concentrations include 0.5, 1, 1.5, 2 or 2.5 weight %.

Due to its explosive properties, GTN intended for pharmaceutical purposes is phlegmatized by the manufacturer, i.e. embedded in a matrix, which reduces the risk posed by the hazardous properties. The matrix can be in liquid or in powder form. For example, GTN is available as a 5% solution in medium-chain triglycerides, such as Miglyol^{®} 812 or Miglyol^{®} 840, or propylene glycol, as a 10% lactose triturate or a 2.25% dilution in glucose. If these GTN concentrates are used for producing the pharmaceutical preparation according to the invention, the agent used for phlegmatization of GTN is regularly contained in the finished product. If GTN is phlegmatized in a volatile solvent like ethanol, the solvent can be lost during the production process.

### Examples

The following examples illustrate various preparations according to the invention and methods of their preparation.

GTN was used as a 5% concentrate in Miglyol^{®} 812. Unless otherwise stated, the optionally pre-granulated excipients were processed into a homogenous mixture with the GTN concentrate.

### Example 1

| Ingredients | Amount [g] |
|---|---|
| Isomalt | 68.35 |
| Xylitol | 25.00 |
| GTN | 0.21 |
| Medium-chain triglycerides * | 3.78 |
| Flavoring agent | 1.61 |
| Caprylic / Capric glycerides ** | 1.01 |
| Total | 99.96 |

| | |
|---|---|
| * Commercial name: Miglyol 812 ** Commercial name: Imwitor 742 | |

The granules according to Example 1 were packaged in the packaging materials referred to in Examples 2 through 5 and stored for 12 weeks at 25°C and 60% relative humidity and at 40°C and 75% relative humidity. As a reference, granules were stored in open dishes and in glass containers.

### Comparative example 2

PET - aluminum - 75 µm LDPE (low-density polyethylene)

### Comparative example 3

PET - aluminum - 40 µm LDPE

### Comparative example 4

PET - aluminum - 50 µm PP (polypropylene)

### Example (according to the invention) 5

PET - aluminum - 50 µm PAN (Barex^{®} 210)

After four and 12 weeks of storage, samples were taken and the GTN content was quantified using HPLC analysis. Thereby a quantity of powder or granules corresponding to a single dose was dissolved in a suitable solvent. GTN was detected using a UV-VIS detector at a wavelength of 225 nm.

The results are shown in the table below:

| Packaging | GTN content in % of initial content following storage at 25 °C | | GTN content in % of initial content following storage at 40 °C | |
|---|---|---|---|---|
| | 4 weeks | 12 weeks | 4 weeks | 12 weeks |
| Open storage | n.m. | n.m. | 60 % | 17 % |
| Comp. example 2 | 72 % | 53 % | 51 % | n.m. |
| Comp. example 3 | 76 % | 66 % | 66 % | n.m. |
| Comp. example 4 | 94 % | 85 % | 78 % | n.m. |
| Example 5 | 97 % | 97 % | 97 % | 95 % |
| Glass | 103 % | 104 % | 102 % | 98 % |

| | | | | |
|---|---|---|---|---|
| n.m. = not measured (The results after four weeks were so poor in the case of some of the film materials that the test was terminated.) | | | | |

The results demonstrate that the GTN containing preparation, when stored in a composite film containing a layer comprising PAN at the side faced with the preparation, especially in a composite film containing an impact-modified acrylonitrile-methylacrylate copolymer as the layer coming into contact with the pharmaceutical product, achieve the same stability as the pharmaceutical preparation when stored in a glass container. By contrast a significant active substance loss occurred, when the GTN containing preparation is packed in composite films comprising a different sealing layer such as LPDE or PP on the side facing the preparation.

The preparation according to the present invention can be supplied as individual dose, e.g. within a stick pack or sachet, wherein the aluminum composite film contains a layer comprising PAN on the side facing the preparation. In addition, it is possible to prepare tablets, mini-tablets or pellets for sublingual administration from granules, if necessary, following the addition of other pharmaceutically acceptable excipients such as fillers, disintegrants, glidants, binders, and lubricants, or to fill the granules into hard gelatin two-piece capsules. Single doses of GTN in form of tablets, pellets or capsules can likewise be filled in stick packs and sachets or packaged in blisters, whose thermoplastic film surface contains a layer comprising PAN on the side facing the preparation. The same applies to single doses of GTN available in the form of oral fast dissolving films, which can be packed into aluminium or thermoplastic composite films carrying a layer comprising PAN at the surface, which is in direct contact with the preparation.

## Claims

1. Solid pharmaceutical preparation, comprising glyceryl trinitrate (GTN), in the form of a single dose for oromucosal or oral administration, which is packaged in a composite film, wherein the composite film contains a layer comprising polyacrylonitrile (PAN) on the side facing the pharmaceutical preparation.

2. Preparation according to claim 1, wherein the composite film is an aluminum composite film.

3. Preparation according to claim 1, wherein the composite film is a thermoplastic composite film.

4. Preparation according to anyone of claims 1 to 3, wherein the layer of the composite film on the side facing the pharmaceutical preparation comprises acrylonitrile-methylacrylate copolymer.

5. Preparation according to claim 4, wherein the layer of the composite film on the side facing the pharmaceutical preparation comprises impact-modified acrylonitrile-methylacrylate copolymer.

6. Preparation according to anyone of the claims 4 or 5, wherein the layer of the composite film on the side facing the pharmaceutical preparation is impact-modified acrylonitrile-methylacrylate copolymer.

7. Preparation according to anyone of claims 1 to 6 **characterized in that** it contains between 0.05 and 2 weight % glyceryl trinitrate.

8. Preparation according to anyone of claims 1 to 7 containing at least one other excipient suitable for sublingual administration selected from fructose, glucose, isomalt, lactose, maltitol, maltose, mannitol, saccharose, sorbitol, trehalose and xylitol, and mixtures thereof.

9. Preparation according to claim 8 containing at least one other excipient suitable for sublingual administration, which is xylitol and/or isomalt at concentrations of 20 to 95 weight %.

10. Preparation according to anyone of the preceding claims containing at least one flavoring agent.

11. Preparation according to anyone of the preceding claims, wherein GTN is formulated in the form of granules.

12. Preparation according to anyone of the preceding claims, which is packaged in the form of a single dose for oromucosal or oral administration.

13. Preparation according to anyone of the preceding claims wherein the single dosage form is contained in a stick pack.

14. Preparation according to anyone of claims 1 to 9, wherein the GTN is formulated in the form of a tablet.

15. Preparation in accordance with anyone of claims 1 to 9, wherein the GTN is formulated in the form of an oral fast dissolving film.

## Patentansprüche

1. Feste pharmazeutische Zubereitung umfassend Glyceryltrinitrat (GTN) in Form einer Einzeldosis für die oromukosale oder orale Verabreichung, die in eine Verbundfolie verpackt ist, wobei die Verbundfolie auf der Seite, die der pharmazeutischen Zubereitung zugewandt ist, eine Schicht enthaltend Polyacrylnitril (PAN) umfasst.

2. Zubereitung gemäß Anspruch 1, wobei die Verbundfolie eine Aluminiumverbundfolie ist.

3. Zubereitung gemäß Anspruch 1, wobei die Verbundfolie eine thermoplastische Verbundfolie ist.

4. Zubereitung gemäß einem der Ansprüche 1 bis 3, wobei die der pharmazeutischen Zubereitung zugewandte Schicht der Verbundfolie Acrylnitrilmethylacrylat-Copolymer umfasst.

5. Zubereitung gemäß Anspruch 4, wobei die der pharmazeutischen Zubereitung zugewandte Schicht der Verbundfolie schlagzäh modifiziertes Acrylnitrilmethylacrylat-Copolymer umfasst.

6. Zubereitung gemäß einem der Ansprüche 4 oder 5, wobei die der pharmazeutischen Zubereitung zugewandte Schicht der Verbundfolie schlagzäh modifiziertes Acrylnitrilmethylacrylat-Copolymer ist.

7. Zubereitung gemäß einem der Ansprüche 1 bis 6, **gekennzeichnet dadurch, dass** sie zwischen 0,05 und 2 Gew.-% Glyceryltrinitrat enthält.

8. Zubereitung gemäß einem der Ansprüche 1 bis 7 enthaltend wenigstens einen weiteren, für die sublinguale Verabreichung geeigneten Hilfsstoff, der ausgewählt ist aus Fruktose, Glukose, Isomalt, Laktose, Maltitol, Maltose, Mannitol, Saccharose, Sorbitol, Trehalose und Xylitol sowie Mischungen davon.

9. Zubereitung gemäß Anspruch 8 enthaltend wenigstens einen weiteren, für die sublinguale Verabreichung geeigneten Hilfsstoff, welcher Xylitol und/oder Isomalt in Konzentrationen von 20 bis 95 Gew.-% ist.

10. Zubereitung gemäß einem der vorhergehenden Ansprüche enthaltend wenigstens einen Geschmacksstoff.

11. Zubereitung gemäß einem der vorhergehenden Ansprüche, wobei GTN in Form eines Granulats formuliert ist.

12. Zubereitung gemäß einem der vorhergehenden Ansprüche, die in Form einer Einzeldosis für die oromukosale oder orale Verabreichung verpackt ist.

13. Zubereitung gemäß einem der vorhergehenden Ansprüche, wobei die Einzeldosis in einem Stickpack enthalten ist.

14. Zubereitung gemäß einem der Ansprüche 1 bis 9, wobei das GTN in Form einer Tablette formuliert ist.

15. Zubereitung gemäß einem der Ansprüche 1 bis 9, wobei GTN in Form eines oral schnell auflösenden Films vorliegt.

## Revendications

1. Composition pharmaceutique solide comprenant du glyceryl trinitrate (GTN), sous la forme d'une dose unique pour administration par voie oromucosale ou par voie orale, qui est emballée dans un film composite, lequel film composite contient une couche comprenant du polyacrylonitrile (PAN) du côté faisant face à la composition pharmaceutique.

2. Composition selon la revendication 1 où le film composite est un film composite en aluminium.

3. Composition selon la revendication 1 où le film composite est un film composite thermoplastique.

4. Composition selon l'une quelconque des revendications 1 à 3 où la couche du film composite du côté faisant face à la composition pharmaceutique comprend un copolymère d'acrylonitrile-methylacrylate.

5. Composition selon la revendication 4 où la couche du film composite du côté faisant face à la composition pharmaceutique comprend un copolymère d'acrylonitrile-methylacrylate modifié par impact.

6. Composition selon l'une quelconque des revendications 4 ou 5, où la couche du film composite du côté faisant face à la composition pharmaceutique comprend un copolymère d'acrylonitrile-methylacrylate modifié par impact.

7. Composition selon l'une quelconque des revendications 1 à 6, **caractérisée en ce qu'**elle contient entre 0.05 et 2 % en masse de glyceryl trinitrate.

8. Composition selon l'une quelconque des revendications 1 à 7 contenant au moins un autre excipient approprié pour une administration par voie sublinguale sélectionné parmi le fructose, le glucose, l'isomalt, le lactose, le maltitol, le maltose, le mannitol, le saccharose, le sorbitol, le trehalose et le xylitol, et leurs mélanges.

9. Composition selon la revendication 8 contenant au moins un autre excipient approprié pour une administration par voie sublinguale, qui est le xylitol et/ou l'isomalt à des concentrations de 20 à 95 % en masse.

10. Composition selon l'une quelconque des revendications précédentes contenant au moins un agent aromatisant.

11. Composition selon l'une quelconque des revendications precedentes où GTN est formulé sous forme de granules.

12. Composition selon l'une quelconque des revendications precedentes qui est emballée sous la forme d'une dose unique pour administration par voie oromucosale ou par voie orale.

13. Composition selon l'une quelconque des revendications precedentes où la forme de dosage unique est contenue dans un sachet stick.

14. Composition selon l'une quelconque des revendications 1 à 9 où le GTN est formulé sous la forme d'un comprimé.

15. Composition selon l'une quelconque des revendications 1 à 9 où le GTN est formulé sous la forme d'un film à dissolution rapide par voie orale.
